# EUROPÄISCHE PATENTSCHRIFT

(11) **EP 2 432 317 B1**
(45) Veröffentlichungstag und Bekanntmachung des Hinweises auf die Patenterteilung: **17.07.2013**
(21) Anmeldenummer: 10720002.4
(22) Anmeldetag: 11.05.2010
(51) Int. Cl.: A61K 8/49, A61K 8/34, A61K 8/37, A61Q 19/00, A01N 43/08, A01N 31/04, A01N 31/14, A01N 43/16

(54) **ZUSAMMENSETZUNG ENTHALTEND SORBITANMONOCAPRYLAT UND ALKOHOL**
COMPOSITION CONTAINING SORBITAN MONOCAPRYLATE AND ALCOHOL
COMPOSITION CONTENANT DU MONOCAPRYLATE DE SORBITANE ET UN ALCOOL

(30) Priorität: 23.05.2009 DE 102009022445
(43) Veröffentlichungstag der Anmeldung: 28.03.2012
(60) Teilanmeldung: 13169070.3
(73) Patentinhaber: Clariant Finance (BVI) Limited, Road Town, Tortola (VG)
(72) Erfinder: KLUG, Peter, 63762 Großostheim (DE); GEHM, Sonja, 65812 Bad Soden am Taunus (DE); KLUTH, Guiseppina, 65779 Kelkheim (DE); SCHERL, Franz-Xaver, 84508 Burgkirchen (DE); PILZ, Maurice, Frederic, 60329 Frankfurt am Main (DE)
(74) Vertreter: Jacobi, Markus Alexander
(86) Internationale Anmeldenummer: PCT/EP2010/002918
(87) Internationale Veröffentlichungsnummer: WO 2010/136120

(56) Entgegenhaltungen:
- EP-A2- 1 813 251
- WO-A2-2010/108738
- JP-A- 51 056 809
- JP-A- 51 068 608
- JP-A- 2003 238 396
- US-A- 3 331 742
- US-A- 4 637 930
- US-A1- 2005 222 276
- DATABASE CA [Online] CHEMICAL ABSTRACTS SERVICE, COLUMBUS, OHIO, US; 29. September 2000 (2000-09-29), Fukushima Noriko: "Water-soluble rinses for dishwashers", XP002643077, Database accession no. 133:209704
- DATABASE CA [Online] CHEMICAL ABSTRACTS SERVICE, COLUMBUS, OHIO, US; 14. August 2008 (2008-08-14), Mori Toshiki: "Transparent cleansers comprising nonionic surfactants", XP002643078, Database accession no. 149:183047
- DATABASE CA [Online] CHEMICAL ABSTRACTS SERVICE, COLUMBUS, OHIO, US; 12. Mai 1984 (1984-05-12), Miura Takeshi, Ishida Katsuo: "Rinsing assistants", XP002643079, Database accession no. 85:96134
- DATABASE CA [Online] CHEMICAL ABSTRACTS SERVICE, COLUMBUS, OHIO, US; 12. Mai 1984 (1984-05-12), Miura Takeshi, Ishida Katsuo: "Rinsing assistants", XP002643080, Database accession no. 85:96140
- DATABASE CA [Online] CHEMICAL ABSTRACTS SERVICE, COLUMBUS, OHIO, US; 18. September 2003 (2003-09-18), Minemura, Takeshi; Kubota, Hiroyuki; Akimaru, Mikuo: "Coenzyme Q10-containing emulsions, and manufacture thereof", XP002643081, Database accession no. 139:202492
- DATABASE GNPD [Online] Mintel; Februar 1999 (1999-02), "Verzorgende Shampoo-Lang Haar", XP002643082, Database accession no. 1927
- BACH M ET AL: "KONSERVIERUNGSMITTEL UND IHRE PRAKTISCHE ANWENDUNG IN KOSMETISCHEN PRODUKTEN", SOFW-JOURNAL SEIFEN, OELE, FETTE, WACHSE, VERLAG FUR CHEMISCHE INDUSTRIE, AUGSBURG, DE, Bd. 116, Nr. 9, 13. Juni 1990 (1990-06-13) , Seiten 345-356, XP000134744, ISSN: 0942-7694

## Beschreibung

Die vorliegende Erfindung betrifft flüssige Zusammensetzungen enthaltend Sorbitanmonocaprylat und Alkohol.

Die Verwendung von Alkoholen zum Konservieren kosmetischer, dermatologischer oder pharmazeutischer Formulierungen und Produkte, beispielsweise von Benzylalkohol oder 1,2-Octandiol, ist bekannt (David Steinberg, Preservatives for Cosmetics, Allured Publishing Corporation, zweite Auflage 2006). Die maximale Einsatzkonzentration der Alkohole ist teils durch gesetzliche Regularien, z. B. in der EU durch den ANNEX VI der Kosmetikrichtlinie, vorgegeben. Zum anderen auch durch den viskositätserniedrigenden Effekt der Alkohole in der Formulierung, wenn diese in höheren Konzentrationen eingesetzt werden. Die Erniedrigung der Viskosität ist hierbei ein unerwünschter Nebeneffekt. Zudem kann es weiterhin zu Phasenseparationen innerhalb der kosmetischen, dermatologischen oder pharmazeutischen Formulierung kommen.

Nicht alle antimikrobiell wirksamen Alkohole sind dermatologisch und toxikologisch unbedenklich, weshalb die verwendete Menge an diesen Alkoholen in der kosmetischen, dermatologischen oder pharmazeutischen Formulierung so dosiert sein sollte, dass die Formulierung gerade ausreichend gegen mikrobiellen Befall geschützt ist. Allerdings reicht oftmals die maximal zulässige oder die maximale sich nicht negativ auf die Formulierung auswirkende Einsatzkonzentration der Alkohole nicht aus, um die kosmetische, dermatologische oder pharmazeutische Formulierung hinreichend gegen mikrobiellen Befall zu schützen.

Um die Gesamtmenge an antimikrobiell wirksamen Alkoholen in der kosmetischen, dermatologischen oder pharmazeutischen Formulierung gering zu halten, bestand die Aufgabe, eine dermatologisch und toxikologisch unbedenkliche Substanz zu finden, die in synergistischer Weise die antimikrobielle Wirkung der antimikrobiell wirksamen Alkohole unterstützt.

Überraschend wurde gefunden, dass das bereits in der Kosmetik als Tensid und emulgierendes Agens bekannte und verwendete Sorbitanmonocaprylat genau diese Bedingungen erfüllt.

Gegenstand der vorliegenden Erfindung sind daher flüssige Zusammensetzungen enthaltend
a) von 5 bis 95 Gew.-%, bevorzugt von 10 bis 90 Gew.-%, besonders bevorzugt von 20 bis 80 Gew.-% und insbesondere bevorzugt von 30 bis 70 Gew.-% Sorbitanmonocaprylat und
b) von 5 bis 95 Gew.-%, bevorzugt von 10 bis 90 Gew.-%, besonders bevorzugt von 20 bis 80 Gew.-% und insbesondere bevorzugt von 30 bis 70 Gew.-% an einem oder mehreren Alkoholen der Formel (1)

   R-OH (1)

   worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können, dadurch gekennzeichnet, daß Komponente b) mindestens 50 Gew.-% an einem oder mehreren Alkoholen der Formel (1) ausgewählt aus aromatischen Alkoholen enthält.

Sorbitanmonocaprylat ist dermatologisch wie toxikologisch auch in sehr hohen Einsatzkonzentrationen unbedenklich und unterstützt in synergistischer Weise die antimikrobielle Wirkung von antimikrobiell wirksamen Alkoholen.

Es wurde weiter gefunden, dass Sorbitanmonocaprylat nicht die Viskosität einer kosmetischen, dermatologischen oder pharmazeutischen Formulierung verringert, sondern sogar im Gegenteil leicht verdickende Eigenschaften besitzt. So können im Vergleich zu den antimikrobiell wirksamen Alkoholen deutlich höhere Mengen an Sorbitanmonocaprylat eingesetzt werden, ohne die Viskosität der kosmetischen, dermatologischen oder pharmazeutischen Formulierung zu erniedrigen oder eine Phasenseparation zu begünstigen.

Die für eine hinreichende Konservierung der kosmetischen, dermatologischen oder pharmazeutischen Formulierung benötigte Einsatzkonzentration von antimikrobiell wirksamen Alkoholen kann in der Kombination mit Sorbitanmonocaprylat signifikant verringert werden. Dadurch reicht oft die Verwendung eines antimikrobiell wirksamen Alkohols zur Konservierung der kosmetischen, dermatologischen oder pharmazeutischen Formulierung aus.

Sorbitanmonocaprylat ist bei Raumtemperatur flüssig und mit den antimikrobiell wirksamen Alkoholen mischbar.

Vorteilhaft an den erfindungsgemäßen flüssigen und daher leicht handhabbaren Zusammensetzungen ist beispielsweise ihre gute Formulierbarkeit.

Vorzugsweise ist der eine oder sind die mehreren Alkohole der Formel (1) ausgewählt aus der Gruppe bestehend aus aromatischen Alkoholen, Alkandiolen und Alkantriolen und besonders bevorzugt ist der eine oder sind die mehreren Alkohole der Formel (1) ausgewählt aus der Gruppe bestehend aus aromatischen Alkoholen und Alkandiolen.

Insbesondere bevorzugt ist der eine oder sind die mehreren Alkohole der Formel (1) ausgewählt aus der Gruppe der Alkohole bestehend aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol und Ethylhexylglycerin, wobei unter den aromatischen Alkoholen wiederum Benzylalkohol und Phenoxyethanol bevorzugt sind und Benzylalkohol besonders bevorzugt ist.

In einer bevorzugten Ausführungsform der Erfindung enthält Komponente b) der erfindungsgemäßen flüssigen Zusammensetzungen 60 bis 100 Gew.-% und bevorzugt 100 Gew.-% an einem oder mehreren Alkoholen der Formel (1) ausgewählt aus aromatischen Alkoholen, wobei der eine oder die mehreren aromatischen Alkohole vorzugsweise aus Benzylalkohol, Phenoxyethanol, Propylene Phenoxyethanol und Phenethylalkohol und besonders bevorzugt aus Benzylalkohol und Phenoxyalkohol ausgewählt sind und es sich bei dem einen oder den mehreren aromatischen Alkoholen insbesondere bevorzugt um Benzylalkohol handelt.

Unter den soeben genannten erfindungsgemäßen flüssigen Zusammensetzungen sind in einer bevorzugten Ausführungsform der Erfindung wiederum diejenigen bevorzugt, wobei Komponente b) neben dem einen oder den mehreren Alkoholen der Formel (1) ausgewählt aus aromatischen Alkoholen einen oder mehrere nichtaromatische Alkohole, vorzugsweise ausgewählt aus der Gruppe der Alkandiole bestehend aus 1,2-Hexandiol, 1,6-Hexandiol, 1,2-Octandiol, 1,2-Decandiol und Ethylhexylglycerin, enthält.

In einer besonders bevorzugten Ausführungsform der Erfindung enthalten die erfindungsgemäßen flüssigen Zusammensetzungen ein oder mehrere weitere Substanzen ausgewählt aus
d) Wasser,
e) antimikrobiellen Wirkstoffen und
f) Hydrotropen,
wobei die antimikrobiellen Wirkstoffe und Hydrotrope zu den Alkoholen der Formel (1) unterschiedlich sind.

Die antimikrobiellen Wirkstoffe der Komponente e) und die Hydrotrope der Komponente f) sind unterschiedlich zu Sorbitanmonocaprylat.

In einer besonders bevorzugten Ausführungsform der Erfindung enthalten die erfindungsgemäßen flüssigen Zusammensetzungen Wasser. In einer hierunter wiederum bevorzugten Ausführungsform der Erfindung ist das Wasser in einer Menge von 0,1 bis 35 Gew.-%, vorzugsweise von 0,1 bis 20 Gew.-% und besonders bevorzugt von 0,5 bis 10 Gew.-% in den erfindungsgemäßen flüssigen Zusammensetzungen enthalten.

In einer weiteren besonders bevorzugten Ausführungsform der Erfindung enthalten die erfindungsgemäßen flüssigen Zusammensetzungen ein oder mehrere antimikrobielle Wirkstoffe, die zu den Alkoholen der Formel (1) unterschiedlich sind.

Diese antimikrobiellen Wirkstoffe sind vorzugsweise ausgewählt aus Piroctone Olamine (2-Aminoethanol-Salz von 1-Hydroxy-4-methyl-6-(2,4,4-trimethylpentyl)-2(1H)-pyridon, Handelsprodukt ist Octopirox^{®}), Parabenen, wie beispielsweise Methylparaben, Ethylparaben, Propylparaben, Isopropylparaben, Butylparaben, Isobutylparaben, Natrium Methylparaben, Natrium Ethylparaben, Natrium Propylparaben, Natrium Isobutylparaben, Natrium Isopropylparaben oder Natrium Butylparaben, organischen Säuren und ihren Salzen, wie beispielsweise Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure, 2,4-Hexandiensäure und/oder ihre Salze wie beispielsweise Natriumbenzoat, Kaliumsorbat oder Salicylate wie z. B. Natriumsalicylat,
Imidazolidinyl Harnstoff, Diazolidinyl Harnstoff, lodopropynyl Butylcarbamat, 2-Bromo-2-Nitropropan-1,3-diol, Cetyltrimethylammoniumchlorid, Cetylpyridiniumchlorid, Benzethoniumchlorid, Diisobutylethoxyethyldimethylbenzylammoniumchlorid, Diisobutyl-phenoxy-ethoxy-ethyl-dimethylbenzylammoniumchlorid, N-Alkyl-N,N-dimethyl-benzyl-ammoniumchlorid, -bromid, -saccharinat, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), 3,4,4'-Trichlorocarbanilid (Triclocarban), Diaminoalkylamid, beispielsweise L-Lysinhexadecylamid, DMDM Hydantoin, Natrium Hydroxymethylglycinat, 2-Hydroxybiphenyl, Chlorbutanulum, 5-Amino-1,3-bis-(2-ethylhexyl)-5-methyl-hexahydropyrimidin, 2,4-Dichlorbenzylalkohol, N-(4-Chlorphenyl-N'-(3,4-dichlorphenyl)harnstoff, 2,4,4'-Trichlor-2'-hydroxydiphenylether, Poly(hexamethylendiguanid)-hydrochlorid, 1,2-Dibrom-2,4-dicyanobutan, 4,4-Dimethyl-1,3-oxazolidin, Isothiazolinonen, wie beispielsweise Methylisothiazolinon oder Methylchloroisothiazolinon und Methylisothiazolinon im molaren Verhältnis von 3:1, Chloroxylenol, Citratschwermetallsalzen, Silberchlorid, Piroctose, insbesondere Zinksalzen, Pyrithionen und deren Schwermetallsalzen, insbesondere Zinkpyrithion, Zinkphenolsulfat, Farnesol, Bifonazole, Butoconazole, Cloconazole, Clotrimazole, Econazole, Enilconazole, Fenticonazole, Fluconazole, Isoconazole, Itraconazole, Ketoconazol, Miconazole, Naftifine, Oxiconazol, Sulconazole, Terbinafine, Terconazole und Tioconazole und Kombinationen dieser Wirksubstanzen.

In einer insbesondere bevorzugten Ausführungsform der Erfindung enthalten die erfindungsgemäßen flüssigen Zusammensetzungen ein oder mehrere antimikrobielle Wirkstoffe, die zu den Alkoholen der Formel (1) unterschiedlich sind, ausgewählt aus Parabenen, vorzugsweise Methylparaben, Ethylparaben, Propylparaben, Butylparaben, Isobutylparaben, Natrium Methylparaben, Natrium Ethylparaben, Natrium Propylparaben, Natrium Isobutylparaben und/oder Natrium Butylparaben,
organischen Säuren und ihren Salzen, vorzugsweise Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und/oder ihre Salze wie beispielsweise Natriumbenzoat, Kaliumsorbat und/oder Natriumsalicylat, Formaldehydabspaltern, vorzugsweise Imidazolidinyl Harnstoff, Diazolidinyl Harnstoff, DMDM Hydantoin und/oder Natrium Hydroxymethylglycinat, halogenierten Konservierungsstoffen, vorzugsweise lodopropynyl Butylcarbamat und/oder 2-Bromo-2-Nitropropan-1,3-diol und
Isothiazolinonen, vorzugsweise Methylisothiazolinon.

In einer außerordentlich bevorzugten Ausführungsform der Erfindung enthalten die erfindungsgemäßen flüssigen Zusammensetzungen ein oder mehrere antimikrobielle Wirkstoffe, die zu den Alkoholen der Formel (1) unterschiedlich sind, ausgewählt aus organischen Säuren und ihren Salzen, vorzugsweise ausgewählt aus Benzoesäure, Sorbinsäure, 3-Acetyl-6-methyl-2[H]-pyran-2,4[3H]-dione (Dehydroacetic acid), p-Methoxybenzoesäure, Ameisensäure, Essigsäure, Propionsäure, Milchsäure, Undecensäure, Salicylsäure, Glykolsäure und deren Salzen.

Sofern die erfindungsgemäßen flüssigen Zusammensetzungen ein oder mehrere antimikrobielle Wirkstoffe, die zu den Alkoholen der Formel (1) unterschiedlich sind, enthalten, sind diese vorzugsweise in einer Menge von 0,5 bis 30 Gew.-% und besonders bevorzugt von 1,0 bis 25 Gew.-% in den erfindungsgemäßen flüssigen Zusammensetzungen enthalten.

In dem Fall, dass die erfindungsgemäßen flüssigen Zusammensetzungen ein oder mehrere weitere antimikrobielle Wirkstoffe ausgewählt aus Salzen von organischen Säuren enthalten, enthalten die erfindungsgemäßen flüssigen Zusammensetzungen vorzugsweise von 2 bis 35 Gew.-%, besonders bevorzugt von 5 bis 20 Gew.-% und insbesondere bevorzugt von 10 bis 15 Gew.-% Wasser.

In einer weiteren besonders bevorzugten Ausführungsform der Erfindung enthalten die erfindungsgemäßen flüssigen Zusammensetzungen ein oder mehrere Hydrotrope, die zu den Alkoholen der Formel (1) unterschiedlich sind. Diese Hydrotrope sind vorzugsweise ausgewählt aus Xylol-, Toluol- und Cumolsulfonat. Cumolsulfonat ist besonders bevorzugt.

Sofern die erfindungsgemäßen flüssigen Zusammensetzungen ein oder mehrere Hydrotrope, die zu den Alkoholen der Formel (1) unterschiedlich sind, enthalten, ist die Menge des einen oder der mehreren dieser Hydrotrope in den erfindungsgemäßen flüssigen Zusammensetzungen vorzugsweise im Bereich von 1 bis 15 Gew.-%, besonders bevorzugt von 4 bis 10 Gew.-% und insbesondere bevorzugt von 6 bis 8 Gew.-%.

In einer weiteren besonders bevorzugten Ausführungsform der Erfindung enthalten die erfindungsgemäßen flüssigen Zusammensetzungen ein oder mehrere weitere Additive.

Diese weiteren Additive sind vorzugsweise ausgewählt aus Antioxidantien und Solubilisatoren.

In einer weiteren besonders bevorzugten Ausführungsform der Erfindung enthalten die erfindungsgemäßen flüssigen Zusammensetzungen ein oder mehrere Antioxidantien.

Die Antioxidantien sind vorzugsweise ausgewählt aus Superoxid-Dismutase, Tocopherol (Vitamin E), Ascorbinsäure (Vitamin C), Aminosäuren (z. B. Glycin, Histidin, Tyrosin, Tryptophan) und deren Derivaten, Imidazolen (z. B. Urocaninsäure) und deren Derivaten, Peptiden wie D,L-Camosin, D-Camosin, L-Carnosin und deren Derivaten (z. B. Anserin), Carotinoiden, Carotinen (z.B. α-Carotin, β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Phytinsäure, Gallensäure, Gallenextrakten, Bilirubin, Biliverdin, EDTA, EGTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin-E-acetat), Vitamin A und Derivaten (Vitamin-A-palmitat), Koniferylbenzoat des Benzoeharzes, Rutinsäure und deren Derivaten, α-Glycosylrutin, Ferulasäure, Furfurylidenglucitol, Camosin, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z.B. ZnO, ZnSO₄) Selen und dessen Derivaten (z. B. Selenmethionin), Stilbenen und deren Derivaten (z. B. Stilbenoxid, trans-Stilbenoxid) und Superoxid-Dismutase und erfindungsgemäß geeigneten Derivaten (Salze, Ester, Ether, Zucker, Nukleotide, Nukleoside, Peptide und Lipide) dieser genannten Stoffe.

Besonders bevorzugte Antioxidantien sind ausgewählt aus öllöslichen Antioxidantien.

Insbesondere bevorzugte Antioxidantien sind ausgewählt aus Tocopherylacetat und EDTA.

Sofern die erfindungsgemäßen flüssigen Zusammensetzungen ein oder mehrere Antioxidantien enthalten, sind diese vorzugsweise in einer Menge von 0,001 bis 30 Gew.-%, besonders bevorzugt von 0,05 bis 20 Gew.-% und insbesondere bevorzugt von 0,1 bis 5 Gew.-% in den erfindungsgemäßen flüssigen Zusammensetzungen enthalten.

In einer weiteren besonders bevorzugten Ausführungsform der Erfindung enthalten die erfindungsgemäßen flüssigen Zusammensetzungen ein oder mehrere Solubilisatoren.

Bevorzugte Solubilisatoren sind Verbindungen ausgewählt aus der Gruppe bestehend aus Ethanol, Propanol, Isopropanol, n-Butanol, iso-Butanol, Butylenglykol, 1,2-Propylenglykol, Polyethylenglykolen mit einer relativen Molekülmasse von 300 bis 2000, insbesondere mit einer relativen Molekülmasse von 300 bis 600, Triacetin (Glycerintriacetat), 1-Methoxy-2-propanol und PEG-4-Laurat (Polyethylenglykol-4-Laurat).

Besonders bevorzugte Solubilisatoren sind ausgewählt aus Butylenglykol und 1,2-Propylenglykol.

Sofern die erfindungsgemäßen flüssigen Zusammensetzungen ein oder mehrere Solubilisatoren enthalten, sind diese vorzugsweise in einer Menge von 1 bis 20 Gew.-% in den erfindungsgemäßen flüssigen Zusammensetzungen enthalten.

In einer weiteren besonders bevorzugten Ausführungsform der Erfindung enthalten die erfindungsgemäßen flüssigen Zusammensetzungen weniger als 5 Gew.-%, vorzugsweise weniger als 3 Gew.-% und besonders bevorzugt weniger als 1 Gew.-% Wasser. In einer insbesondere bevorzugten Ausführungsform der Erfindung enthalten die erfindungsgemäßen flüssigen Zusammensetzungen kein Wasser, d. h. sie sind wasserfrei.

In einer weiteren besonders bevorzugten Ausführungsform der Erfindung bestehen die erfindungsgemäßen flüssigen Zusammensetzungen aus
a) Sorbitanmonocaprylat und
b) einem oder mehreren gemäß Anspruch 1 gewählten Alkoholen der Formel (1).

In einer weiteren besonders bevorzugten Ausführungsform der Erfindung bestehen die erfindungsgemäßen flüssigen Zusammensetzungen aus
a) Sorbitanmonocaprylat,
b) einem oder mehreren gemäß Anspruch 1 gewählten Alkoholen der Formel (1) und
d) Wasser.

In einer weiteren besonders bevorzugten Ausführungsform der Erfindung bestehen die erfindungsgemäßen flüssigen Zusammensetzungen aus
a) Sorbitanmonocaprylat,
b) einem oder mehreren gemäß Anspruch 1 gewählten Alkoholen der Formel (1) und
e) einem oder mehreren antimikrobiellen Wirkstoffen, die zu den Alkoholen der Formel (1) unterschiedlich sind.

In einer weiteren besonders bevorzugten Ausführungsform der Erfindung bestehen die erfindungsgemäßen flüssigen Zusammensetzungen aus
a) Sorbitanmonocaprylat,
b) einem oder mehreren gemäß Anspruch 1 gewählten Alkoholen der Formel (1),
d) Wasser und
e) einem oder mehreren antimikrobiellen Wirkstoffen, die zu den Alkoholen der Formel (1) unterschiedlich sind.

Vorzugsweise besitzen die erfindungsgemäßen flüssigen Zusammensetzungen ein klares Aussehen.

Die erfindungsgemäßen flüssigen Zusammensetzungen sind in vorteilhafter Weise geeignet zum Konservieren kosmetischer, dermatologischer oder pharmazeutischer Produkte.

Weiterer Gegenstand der Erfindung ist daher die Verwendung einer erfindungsgemäßen flüssigen Zusammensetzung zum Konservieren von kosmetischen, dermatologischen oder pharmazeutischen Produkten, vorzugsweise von Cremes, Cremegelen, Lotionen, Shampoos, Duschbädem, Deodorantien, Antiperspirantien, Feuchttüchem (wet wipes), Sonnenschutzformulierungen oder dekorativen Kosmetikartikeln. In einer bevorzugten Ausführungsform der Erfindung werden kosmetische, dermatologische oder pharmazeutische Formulierungen konserviert.

Die erfindungsgemäßen flüssigen Zusammensetzungen sind des Weiteren in vorteilhafter Weise geeignet zur Herstellung von kosmetischen, dermatologischen oder pharmazeutischen Produkten, vorzugsweise von kosmetischen, dermatologischen oder pharmazeutischen Formulierungen.

Weiterer Gegenstand der vorliegenden Erfindung ist daher die Verwendung einer erfindungsgemäßen flüssigen Zusammensetzung zur Herstellung von kosmetischen, dermatologischen oder pharmazeutischen Produkten und vorzugsweise von kosmetischen, dermatologischen oder pharmazeutischen Formulierungen.

Unter dem Begriff "kosmetische, dermatologische oder pharmazeutische Produkte" werden im Rahmen der vorliegenden Erfindung beispielsweise entsprechende Formulierungen verstanden.

Bei den kosmetischen, dermatologischen oder pharmazeutischen Produkten kann es sich beispielsweise um wässrige, wässrig-alkoholische, wässrig-tensidische oder alkoholische Mittel oder um Mittel auf Ölbasis, inklusive Mittel auf Ölbasis in wasserfreier Form oder um Emulsionen, Suspensionen oder Dispersionen handeln und zwar in Form von Fluids, Schäumen, Sprays, Gelen, Mousse, Lotionen, Cremes, Pudern oder Feuchttüchem (Wet Wipes).

In einer bevorzugten Ausführungsform der Erfindung werden die erfindungsgemäßen flüssigen Zusammensetzungen zum Konservieren von Feuchttüchem verwendet. Hierbei kann es sich bei der auf das textile Gewebe aufgetragenen, zu konservierenden Formulierung um eine Emulsion, insbesondere eine O/W Emulsion, aber auch um eine tensidische Formulierung oder ein öliges Mittel handeln.

In einer weiteren bevorzugten Ausführungsform der Erfindung werden die erfindungsgemäßen flüssigen Zusammensetzungen zum Konservieren von Emulsionen verwendet.

Bei den Emulsionen kann es sich sowohl um Wasser-in-Öl-Emulsionen als auch Öl-in-Wasser-Emulsionen, Mikroemulsionen, Nanoemulsionen und multiple Emulsionen handeln. Die Herstellung der Emulsionen kann in bekannter Weise, d. h. beispielsweise durch Kalt-, Heiß-, Heiß/Kalt- oder PIT-Emulgierung erfolgen. Eine besonders bevorzugte Ausführungsform der Erfindung sind selbstschäumende, schaumförmige, nachschäumende oder schäumbare Emulsionen und Mikroemulsionen.

Weiterer Gegenstand der vorliegenden Erfindung ist die Verwendung von Sorbitanmonocaprylat zur Verbesserung der antimikrobiellen Wirksamkeit von Alkoholen der Formel (1)

R-OH (1)

worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können.

Die Herstellung der erfindungsgemäßen flüssigen Zusammensetzungen kann beispielsweise durch Zusammengeben der einzelnen Komponenten, gegebenenfalls unter leichtem Erwärmen auf ca. 50 °C, erfolgen.

Die nachfolgenden Beispiele und Anwendungen sollen die Erfindung näher erläutern, ohne sie jedoch darauf zu beschränken. Bei allen Prozentangaben handelt es sich um Gewichtsprozent (Gew.-%), sofern nicht explizit anders angegeben.

### Beispiele:

### I) Erfindungsgemäße flüssige Zusammensetzungen

### Beispiele 1 - 12

Zusammensetzungen bestehend aus
1) 50 % Sorbitanmonocaprylat, 50 % Benzylalkohol
2) 50 % Sorbitanmonocaprylat, 50 % Phenoxyethanol
3) 60 % Sorbitanmonocaprylat, 40 % 1,2-Octandiol
4) 40 % Sorbitanmonocaprylat, 30 % Ethylhexylglycerin, 30 % 1,2-Hexandiol
5) 50 % Sorbitanmonocaprylat, 50 % Ethylhexylglycerin
6) 45 % Sorbitanmonocaprylat, 30 % Benzylalkohol, 20 % 1,6-Hexandiol, 5 % Wasser
7) 65 % Sorbitanmonocaprylat, 25 % Phenoxyethanol, 10 % Benzoesäure
8) 70 % Sorbitanmonocaprylat, 30 % 1,2-Decandiol
9) 33 % Sorbitanmonocaprylat, 33 % Benzylalkohol, 34 % 1,2-Octandiol
10) 50 % Sorbintanmonocaprylat, 30 % Phenoxyethanol, 20 % Ethylhexylglycerin
11) 40 % Sorbitanmonocaprylat, 15 % 1,2 Hexandiol, 20 % Ethylhexylglycerin, 20 % Dehydroacetic acid, 5 % Wasser
12) 20 % Sorbitanmonocaprylat, 40 % 1,2-Octandiol, 15 % Phenoxyethanol, 15 % Kaliumsorbat, 10 % Wasser

Die Herstellung der Zusammensetzungen der Beispiele 1 bis 12 erfolgte, indem die einzelnen Komponenten unter Rühren nacheinander am Fingerrührer bei Rührgeschwindigkeiten von 200-300 Umdrehungen/Minute vermengt wurden. Im Falle der Zugabe von organischen Säuren wurde die Zusammensetzung auf etwa 50 °C erwärmt, um eine homogene Mischung zu erhalten.

### II) Synergismus zwischen Sorbitanmonocaprylat und Alkoholen der Formel (1)

Berechnung des Synergistischen Effekts nach der Formel Qₐ/Q_{A} + Q_{b}/Q_{B} = SE (Synergistischer Effekt, nach F.C. Kull et al., Applied Microbiology 1961, 9, 538), wobei Qₐ = Minimale Hemmkonzentration der Verbindung A in % in der eingesetzten Mischung, Q_{A} = Minimale Hemmkonzentration der reinen Substanz A, Q_{b} = Minimale Hemmkonzentration der Verbindung B in % in der eingesetzten Mischung und Q_{B} = Minimale Hemmkonzentration der reinen Substanz B. Bewertung des Synergistischen Effekts: wird ein SE > 1 erhalten, so liegt eine antagonistische Wirkung vor, ist SE = 1, so verhalten sich die Verbindungen gegenüber einander neutral und ist SE < 1 liegt ein synergistischer Effekt vor.

### Beispiel 13

Es wurden Mischungen aus Sorbitanmonocaprylat und Phenoxyethanol oder Benzylalkohol gemäß den Beispielen 1) und 2) untersucht. Die Ergebnisse bezüglich des Synergismus sind in den Tabellen A und B aufgeführt. Mit "MIC Blend" ist dabei die Minimale Hemmkonzentration des jeweiligen Blends bezeichnet.

**Tabelle A Ergebnisse der Untersuchung einer Mischung aus Sorbitanmonocaprylat und Phenoxyethanol im Gewichtsverhältnis 50:50 gemäß Beispiel 2) (Blend)**

| | MIC Blend | Sorbitanmonocaprylat | | Phenoxyethanol | | SE |
|---|---|---|---|---|---|---|
| | | Qₐ | Q_{A} | Q_{b} | Q_{B} | |
| Staphylococcus aureus | 0,063 | 0,0315 | 0,25 | 0,0315 | 0,125 | 0,378 |
| Aspergillus brasiliensis | 0,008 | 0,004 | 0,016 | 0,004 | 0,032 | 0,375 |

**Tabelle B Ergebnisse der Untersuchung einer Mischung aus Sorbitanmonocaprylat und Benzylalkohol im Gewichtsverhältnis 50:50 gemäß Beispiel 1) (Blend)**

| | MIC Blend | Sorbitanmonocaprylat | | Phenoxyethanol | | SE |
|---|---|---|---|---|---|---|
| | | Qₐ | Q_{A} | Q_{b} | Q_{B} | |
| Staphylococcus aureus | 0,032 | 0,016 | 0,25 | 0,016 | 0,25 | 0,128 |
| Aspergillus brasiliensis | 0,008 | 0,004 | 0,016 | 0,004 | 0,063 | 0,313 |

### III) Kosmetische Formulierungen enthaltend erfindungsgemäße flüssige Zusammensetzungen

Von jeder der im Folgenden aufgeführten kosmetischen Formulierungen A - M wurden jeweils 12 verschiedene Formulierungen hergestellt. Und zwar wurde jede kosmetische Formulierung A - M jeweils unter Verwendung der einzelnen erfindungsgemäßen flüssigen Zusammensetzungen der Beispiele 1 bis 12 hergestellt.

### Beispiel A - Shampoo

| | | | |
|---|---|---|---|
| A | Genapol^{®} LRO Paste *Sodium Laureth Sulfate* | Clariant | 13,70 % |
| | Genagen^{®} KB Coco *Betaine* | Clariant | 6,00 % |
| | Water | | ad 100 % |
| | | | |
| B | Sodium Chloride | | 1,50 % |
| | | | |
| C | Erfindungsgemäßer Blend 1-12 | Clariant | 1,50 % |
| | | | |
| D | Citric Acid (10 % in water) | | 0,08 % |

### Herstellung:

I Mische die Komponenten von A
II Gebe B unter Rühren zu I
III Gebe C zu II
IV Passe den pH-Wert auf ungefähr 7 an

### Beispiel B - Gesichtsreiniger

| | | | |
|---|---|---|---|
| A | Genapol^{®} LRO liquid | Clariant | 11,10 % |
| | *Sodium Laureth Sulfate* | | |
| | Parfume | | q.s. |
| | | | |
| B | Water | | ad 100 % |
| | Genagen^{®} 3SB | Clariant | 23,30 % |
| | Coco *Betaine, Sodium Cocoyl Isethionate, Sodium Methyl Cocoyl Taurate* | | |
| | Dyestuff solution | | q.s. |
| | | | |
| C | Erfindungsgemäßer Blend 1-12 | Clariant | 1,20 % |
| D | Citric Acid | | q.s. |

### Herstellung:

I Mische die Komponenten A
II Gebe die Komponenten von B nach einander in I
III Gebe C zu II unter Rühren
IV Falls gewünscht, passe den pH-Wert mit C an

### Beispiel C - Aftershave Gel

| | | | |
|---|---|---|---|
| A | Emulsogen^{®} HCU | Clariant | 1,50 % |
| | *Undeceth-8 (and) PEG-40 Hydrogenated Castor Oil* | | |
| | | | |
| B | Tocopherolacetat | | 0,20 % |
| | Menthol | | 0,20 % |
| | | | |
| C | Ethanol | | 30,00 % |
| D | Water | | ad 100 % |
| | Allantoin | Clariant | 0,20 % |
| | *Allantoin* | | |
| | Polyglykol 400 | Clariant | 3,00 % |
| | *PEG-8* | | |
| | Polyglykol 35000 | Clariant | 1,00 % |
| | *PEG-800* | | |
| | Erfindungsgemäßer Blend 1-12 | Clariant | 2,00 % |
| | | | |
| E | Aristoflex^{®} AVC | Clariant | 1,00 % |
| | *Ammonium Acryloyldimethyltaurafe*/*VP Copolymer* | | |

### Herstellung:

I Mische A und B und rühre für etwa 5 Minuten
II Gebe C zu I und rühre bis die Lösung klar ist
III Gebe die Komponenten von D nach einander zu II
IV Gebe E zu I und rühre bis eine homogene Formulierung erhalten wird

### Beispiel D - Anti-Ageing Gesichtscreme

| | | | |
|---|---|---|---|
| A | Genapol^{®} T 250 | Clariant | 1,50% |
| | *Cetereth-25* | | |
| | Genapol^{®} DAT | Clariant | 2,00 % |
| | *PEG-150 Polyglyceryl-2 Tristearate and PEG-6 Caprylic*/*Capric Glyceride* | | |
| B | Water | | ad 100 % |
| | | | |
| C | Aristoflex^{®} AVC | Clariant | 2,00 % |
| | *Ammonium Acryloyldimethyltaurate*/*VP Copolymer* | | |
| D | Glycolic Acid 30 % * | | 6,00 % |
| | Erfindungsgemäßer Blend 1-12 | Clariant | 1,80 % |

| | | | |
|---|---|---|---|
| * mit NaOH auf pH 4 eingestellt (Gehalt basiert auf freier Glykolsäure) | | | |

### Herstellung:

I Löse A in B unter Rühren und leichtem Erwärmen
II Gebe C zu I und rühre bis das entstehende Gel frei von Klümpchen ist
III Gebe die Komponenten von D zu II und rühre bis die Formulierung homogen ist

### Beispiel E - Emulsion für Baby Wet Wipes

| | | | |
|---|---|---|---|
| A | Propylene Glycol | | 3,00 % |
| | Erfindungsgemäßer Blend 1-12 | Clariant | 2,00 % |
| | | | |
| | Emulsogen^{®} HCO 040 | Clariant | 1,00 % |
| | *PEG-40 Hydrogenated Castor Oil* | | |
| | Parfume | | 0,20 % |
| | | | |
| B | Hostaphat^{®} KL 340 D | Clariant | 1,50 % |
| | *Trilaureth-4 Phosphate* | | |
| | Velsan^{®} CCT | Clariant | 0,80 % |
| | *Caprylic*/*Capric Triglyceride* | | |
| | | | |
| C | Water | | ad 100 % |
| | Tetrasodium EDTA | | 0,10 % |
| | | | |
| D | Aristoflex^{®} BLV | Clariant | 0,20 % |
| | *Ammonium Acryloyldimethyltaurate*/*Beheneth- 25 Methacrylate Crosspolymer* | | |
| E | Citric Acid | | q.s. |

### Herstellung:

I Löse die Komponenten von A
II Gebe die Komponenten von B nach einander unter Rühren zu I
III Mische die Komponenten von C
IV Gebe D zu II
V Gebe unter Rühren III zu IV
VI Passe den pH-Wert mit E auf etwa pH 6 an

### Beispiel F - O/W Body Lotion

| | | | |
|---|---|---|---|
| A | Velsan^{®} CCT | Clariant | 3,50 % |
| | *Caprylic*/*Capric Triglyceride* | | |
| | Myristyl Myristate | | 2,50 % |
| | Cetearyl Alcohol | | 2,00 % |
| | Glyceryl Stearate Citrate | | 1,00 % |
| | Octyldodecanol | | 1,00 % |
| | | | |
| B | Aristoflex^{®} AVC | Clariant | 0,60 % |
| | *Ammonium Acryloyldimethyltaurate*/*VP Copolymer* | | |
| | | | |
| C | Water | | ad 100 % |
| | Glycerin | | 7,50 % |
| | | | |
| D | Ethanol | | 3,00 % |
| | Dimethicone | | 3,00 % |
| | Tocopheryl Acetate | | 1,00 % |
| | Aloe Barbadensis | | 1,00 % |
| | Erfindungsgemäßer Blend 1-12 | Clariant | 2,00 % |
| | | | |
| E | Sodium Hydroxide | | q.s. |

### Herstellung:

I Schmelze die Komponenten von A bei etwa 70 °C
II Mische die Komponenten von C und erhitze die Mischung auf etwa 70 °C
III Gebe B zu I wenn I vollständig geschmolzen ist
IV Gebe II zu III
V Bei 35 °C gebe die Komponenten von D zu IV
VI Stelle den pH-Wert mit E auf etwa pH 6,0-6,5 ein

### Beispiel G - Antiperspirant

| | | | |
|---|---|---|---|
| A | Locron^{®} L | Clariant | 30,00 % |
| | *Aluminum Chlorohydrate* | | |
| | Water | | ad 100 % |
| | Polyglykol 400 | Clariant | 3,00 % |
| | *PEG-8* | | |
| | Ethanol | | 17,00 % |
| | Dyestuff solution | | q.s. |
| | Erfindungsgemäßer Blend 1-12 | Clariant | 1,00 % |
| | Fragrance | | 0,30 % |
| | | | |
| B | Tylose^{®} H 4000 G4 | | 2,50 % |
| | *Hydroxyethlycellulose* | | |

### Herstellung:

I Mische die Komponenten von A
II Gebe B unter ständigem Rühren zu I. Rühre so lange weiter, bis die Viskosität ihren Endpunkt erreicht hat und die Formulierung homogen ist.

### Beispiel H - Cremespülung

| | | | |
|---|---|---|---|
| A | Genamin^{®} DSAP | Clariant | 2,50 % |
| | *Distearyldimonium Chloride* | | |
| | Genamin^{®} CTAC | Clariant | 3,00 % |
| | *Cetrimonium Chloride* | | |
| | Hostacerin^{®} T- 3 | Clariant | 1,50 % |
| | *Ceteareth-* 3 | | |
| | Cetyl Alcohol | | 3,00 % |
| | | | |
| B | Water | | ad 100 % |
| | Erfindungsgemäßer Blend 1-12 | Clariant | 1,00 % |
| | | | |
| C | Fragrance | | 0,30 % |
| | Dyestuff solution | | q.s. |

### Herstellung:

I Schmelze A bei etwa 75 °C
II Erhitze B auf etwa 75 °C
III Gebe II unter Rühren zu I und rühre bis zum Abkühlen auf 30 °C
IV Bei etwa 30 °C gebe C unter Rühren zu II

Beispiel I - Haarstyling Gel

| | | | |
|---|---|---|---|
| A | Sorbitol | | 5,00 % |
| | Genamin^{®} PQ 43 | Clariant | 0,30 % |
| | Polyquatemium - 43 | | |
| | | | |
| B | Water | | ad 100 % |
| | | | |
| C | Aristoflex^{®} HMB | Clariant | 2,00 % |
| | *Ammonium Acryloyldimethyltaurate*/*Beheneth- 25* | | |
| | *Methacrylate Crosspolymer* | | |
| | | | |
| D | Aminomethyl Propanol | | 0,30 % |
| | Aristoflex^{®} A 60 | Clariant | 5,00 % |
| | *VA*/*Crotonates Copolymer* | | |
| | Emulsogen^{®} HCO 040 | Clariant | 4,00 % |
| | *PEG-40 Hydrogenated Castor Oil* | | |
| | | | |
| E | Fragrance | | 0,20 % |
| | Erfindungsgemäßer Blend 1-12 | Clariant | 1,00 % |
| | Dyestuff solution | | q.s. |
| | Timiron Diamond Cluster MP- 149 | | q.s. |
| | *Mica (and) Titanium Dioxide (for EU: CI 77891)* | | |

### Herstellung:

I Mische die Komponenten von A
II Gebe B zu I
III Quelle C in II unter Rühren auf
IV Gebe die Komponenten von D eine nach der anderen zu
V Gebe die Komponenten von E eine nach der anderen zu IV

Beispiel J - Make Up Entferner

| | | | |
|---|---|---|---|
| A | Velsan^{®} P8-3 | Clariant | 5,00 % |
| | *Isopropyl C12-15 Pareth-9 Carboxylate* | | |
| | | | |
| B | Hostapon^{®} KCG | Clariant | 2,30 % |
| | *Sodium Cocoyl Glutamate* | | |
| | Genagen^{®} CAB | Clariant | 3,00 % |
| | *Cocamidopropyl Betaine* | | |
| | Genapol^{®} LA 070 | Clariant | 2,00 % |
| | *Laureth-7* | | |
| | Water | | ad 100 % |
| | Allantoin | Clariant | 0,30 % |
| | *Allantoin* | | |
| | Aristoflex^{®} PEA | Clariant | 1,00 % |
| | *Polypropylene Terephthalate* | | |
| | 1,6 Hexanediol | | 2,00 % |
| | 1,2 Propanediol | | 2,00 % |
| | Polyglykol 400 | Clariant | 2,00 % |
| | *PEG-8* | | |
| | Panthenol | | 0,50 % |
| | Lutrol F 127 | | 3,00 % |
| | *Poloxamer 407* | | |
| | Erfindungsgemäßer Blend 1-12 | Clariant | 1,70 % |

### Herstellung:

I Rühre die Komponenten von B nach einander in A und rühre bis eine klare Lösung erhalten wird

### Beispiel K - Lippenglanz

| | | | |
|---|---|---|---|
| A | Versagel^{®} ME 1600 | | ad 100 % |
| | *Hydrogenated polyisobutene (and)* | | |
| | *Ethylene*/*Propylene*/ *Styrene Copolymer (and)* | | |
| | *Buylene*/*Ethylene*/*Styrene Copolymer* | | |
| | SilCare^{®} Silicone 31 M50 | Clariant | 7,00 % |
| | *Caprylyl Trimethicone* | | |
| | SilCare^{®} Silicone 41 M65 | Clariant | 3,00 % |
| | *Stearyl Dimethicone* | | |
| | Jojoba Oil | | 2,60 % |
| | Velsan CCT | Clariant | 1,00 % |
| | *Capric*/*Caprylic Triglycerides* | | |
| | Isopropyl Myristate | | 7,40 % |
| | | | |
| B | Gemtone^{®} Tan Opal | | 1,00 to 5,00 % |
| | *Mica and Iron Oxide and TiO₂* | | |
| | Lake - Color | | q.s. |
| | | | |
| C | Erfindungsgemäßer Blend 1-12 | Clariant | 0,50 % |
| | | | |
| D | Parfum | | q.s. |

### Herstellung:

I Erhitze die Komponenten von A auf etwa 80-85 °C und rühre so lange, bis eine homogene Mischung erhalten wird. Lasse diese Mischung auf 70-75 °C abkühlen
II Gebe B und C nacheinander unter Rühren zu I und rühre, bis alle Bestandteile gelöst sind
III Lasse bis auf 45 °C abkühlen und gebe D zu II, dann fülle die Formulierung in die Gussformen

### Beispiel L - Shimmering Bronze Gel

| | | | |
|---|---|---|---|
| A | Water | | ad 100 % |
| | | | |
| B | Glycerin | | 5,00 % |
| | Polyglykol 35000 S | Clariant | 0,50 % |
| | *PEG-800* | | |
| | Allantoin | Clariant | 0,20 % |
| | *Allantoin* | | |
| C | Aristoflex^{®} AVC | Clariant | 0,60 % |
| | *Ammonium Acryloyldimethyltaurate*/*VP Copolymer* | | |
| | Biron MTU | | 3,00 % |
| | *Bismuth Oxychloride* | | |
| | Flamenco Ultra Silk | | 4,00 % |
| | *Titanium Oxide (and) Mica* | | |
| | Flamenco Sparcle Gold | | 7,00 % |
| | *Mica (and) Iron Oxide (and) Titanium Oxide* | | |
| | Cloisonné Satin Bronze | | 5,00 % |
| | *Iron Oxide (and) Mica* | | |
| | Gemtone Sunstone | | 2,00 % |
| | *Mica (and) Iron Oxide (and) Titanium Oxide* | | |
| | Desert Reflections Canyon Sunset | | 2,00 % |
| | *Mica (and) Iron Oxide (and) Titanium Oxide (and) Tin Oxide* | | |
| | SilCare^{®} Silicone WSI | Clariant | 1,00 % |
| | *proposed INCI: Glyceryl Carboxy Amodimethicone* | | |
| | | | |
| D | Fragrance | | q.s. |
| | Erfindungsgemäßer Blend 1-12 | Clariant | 1,80 % |

### Herstellung:

I Mische die Komponenten von B und löse sie unter Rühren in A
II Mische die Komponenten von C und gebe unter leichtem Rühren zu I
III Rühre mit höherer Umdrehungszahl (etwa 200 - 250 Umdrehungen/Minute) für etwa zwei Stunden oder bis ein homogenes Gel erhalten wird
IV Gebe D unter Rühren zu III

### Beispiel M - Sonnencreme

| | | | |
|---|---|---|---|
| A | SilCare^{®} Silicone WSI | Clariant | 2,00 % |
| | *proposed INCI: Glyceryl Carboxy Amodimethicone* | | |
| | SilCare^{®} Silicone 41 M65 | Clariant | 1 ,00 % |
| | *Stearyl Dimethicone* | | |
| | Dow Corning^{®}246 | | 11,00 % |
| | *Cyclopentasiloxane*/*Cyclohexasiloxane* | | |
| | Titandioxid UV Titan M 262 | | 10,00 % |
| | *Titanium Dioxide*/*Dimethicone* | | |
| | Solaveil CT-100 | | 10,00 % |
| | *C12-15 Alkyl Benzoate*/*Titanium* | | |
| | *Dioxide*/*Aluminium Stearate*/*Polyhydroxystearic Acid*/*Alumina* | | |
| | Z-Cote HP1 | | 8,00 % |
| | *Zinc Oxide* | | |
| | Butylene Glycol | | 3,00 % |
| | Hostacerin^{®} DGI | Clariant | 3,00 % |
| | *Polyglyceryl-2 Sesquiisostearate* | | |
| | Tegosoft^{®} TN | | 2,00 % |
| | *C12-15 Alkyl Benzoate* | | |
| | Cetiol^{®} 868 | | 2,00 % |
| | *Ethylhexylstearate* | | |
| | | | |
| B | Water | | ad 100 % |
| | Glycerin | | 5,00 % |
| | Ginko Biloba Extract | | 0,70 % |
| | Polyglycose | | 0,20 % |
| | Disodium EDTA | | 0,20 % |
| | Citric Acid | | 0,10 % |
| | Glycerin | | 5,00 % |
| | Ginko Biloba Extract | | 0,70 % |
| C | Tocopheryl Acetate | | 1,00 % |
| | Erfindungsgemäßer Blend 1-12 | Clariant | 1,80 % |
| | Sodium Chloride | | 1,00 % |
| | Aluminium Hydroxide | | 0,30 % |

### Herstellung:

I Schmelze A bei etwa 80 °C
II Erwärme B auf etwa 80 °C
III Bei einer Rührgeschwindigkeit von etwa 300 Umdrehungen/Minute gebe II zu I. Erhöhe die Rührgeschwindigkeit nach und nach auf 500 Umdrehungen/Minute und behalte bis zum Ende der Formulierungsarbeit diese Geschwindigkeit bei. Lasse die Mischung auf 35 °C abkühlen
IV Bei 35 °C gebe C unter Rühren zu III und lasse auf Raumtemperatur abkühlen

Die erfindungsgemäßen Zusammensetzungen der Beispiele 1 - 12 tragen in den kosmetischen Formulierungen A - M zur Erhöhung der Biostabilität bei.

## Patentansprüche

1. Flüssige Zusammensetzung enthaltend
a) von 5 bis 95 Gew.-%, bevorzugt von 10 bis 90 Gew.-%, besonders bevorzugt von 20 bis 80 Gew.-% und insbesondere bevorzugt von 30 bis 70 Gew.-% Sorbitanmonocaprylat und
b) von 5 bis 95 Gew.-%, bevorzugt von 10 bis 90 Gew.-%, besonders bevorzugt von 20 bis 80 Gew.-% und insbesondere bevorzugt von 30 bis 70 Gew.-% an einem oder mehreren Alkoholen der Formel (1)
R-OH (1)
worin R ein Rest bestehend aus Kohlenstoff-, Wasserstoff- und gegebenenfalls Sauerstoffatomen mit 5-12, vorzugsweise 6-11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können, **dadurch gekennzeichnet, dass** Komponente b) mindestens 50 Gew.-% an einem oder mehreren Alkoholen der Formel (1) ausgewählt aus aromatischen Alkoholen enthält.

2. Flüssige Zusammensetzung gemäß Anspruch 1, **dadurch gekennzeichnet, dass** der eine oder die mehreren Alkohole der Formel (1) ausgewählt sind aus der Gruppe bestehend aus aromatischen Alkoholen, Alkandiolen und Alkantriolen und vorzugsweise ausgewählt sind aus der Gruppe bestehend aus aromatischen Alkoholen und Alkandiolen.

3. Flüssige Zusammensetzung gemäß Anspruch 1 oder 2, **dadurch gekennzeichnet, dass** der eine oder die mehreren Alkohole der Formel (1) ausgewählt sind aus der Gruppe der Alkohole bestehend aus Benzylalkohol, Phenoxyethanol, Propylene Phenoxyethanol, Phenethylalkohol, 1,2-Pentandiol, 1,2-Hexandiol, 1,6-Hexandiol, 1,2-Octandiol, 1,2-Decandiol, Methylpropandiol und Ethylhexylglycerin, wobei unter den aromatischen Alkoholen wiederum Benzylalkohol und Phenoxyalkohol bevorzugt sind und Benzylalkohol besonders bevorzugt ist.

4. Flüssige Zusammensetzung gemäß einem oder mehreren der Ansprüche 1 bis 3, **dadurch gekennzeichnet, dass** Komponente b) 60 bis 100 Gew.-% und vorzugsweise 100 Gew.-% an einem oder mehreren Alkoholen der Formel (1) ausgewählt aus aromatischen Alkoholen enthält, der eine oder die mehreren aromatischen Alkohole vorzugsweise aus Benzylalkohol, Phenoxyethanol, Propylene Phenoxyethanol und Phenethylalkohol und besonders bevorzugt aus Benzylalkohol und Phenoxyalkohol ausgewählt sind und es sich bei dem einen oder den mehreren aromatischen Alkoholen insbesondere bevorzugt um Benzylalkohol handelt.

5. Flüssige Zusammensetzung gemäß einem oder mehreren der Ansprüche 1 bis 4, **dadurch gekennzeichnet, dass** Komponente b) neben dem einen oder den mehreren Alkoholen der Formel (1) ausgewählt aus aromatischen Alkoholen einen oder mehrere nichtaromatische Alkohole, vorzugsweise ausgewählt aus der Gruppe der Alkandiole bestehend aus 1,2-Hexandiol, 1,6-Hexandiol, 1,2-Octandiol, 1,2-Decandiol und Ethylhexylglycerin, enthält.

6. Flüssige Zusammensetzung gemäß einem oder mehreren der Ansprüche 1 bis 5, **dadurch gekennzeichnet, dass** sie ein oder mehrere weitere Substanzen ausgewählt aus
d) Wasser,
e) antimikrobiellen Wirkstoffen und
f) Hydrotropen
enthält, wobei die antimikrobiellen Wirkstoffe und Hydrotrope zu den Alkoholen der Formel (1) unterschiedlich sind.

7. Flüssige Zusammensetzung gemäß einem oder mehreren der Ansprüche 1 bis 6, **dadurch gekennzeichnet, dass** sie ein oder mehrere antimikrobielle Wirkstoffe, die zu den Alkoholen der Formel (1) unterschiedlich sind, in einer Menge von 0,5 bis 30 Gew.-% und bevorzugt von 1,0 bis 25 Gew.-% enthält.

8. Flüssige Zusammensetzung gemäß einem oder mehreren der Ansprüche 1 bis 7, **dadurch gekennzeichnet, dass** sie ein oder mehrere weitere Additive enthält.

9. Flüssige Zusammensetzung gemäß einem oder mehreren der Ansprüche 1 bis 8, **dadurch gekennzeichnet, dass** sie ein klares Aussehen besitzt.

10. Verwendung einer flüssigen Zusammensetzung nach einem oder mehreren der Ansprüche 1 bis 9 zum Konservieren von kosmetischen, dermatologischen oder pharmazeutischen Produkten, vorzugsweise von Cremes, Cremegelen, Lotionen, Shampoos, Duschbädern, Deodorantien, Antiperspirantien, Feuchttüchem (wet wipes), Sonnenschutzformulierungen oder dekorativen Kosmetikartikeln.

11. Verwendung einer flüssigen Zusammensetzung nach einem oder mehreren der Ansprüche 1 bis 9 zur Herstellung von kosmetischen, dermatologischen oder pharmazeutischen Produkten, vorzugsweise von kosmetischen, dermatologischen oder pharmazeutischen Formulierungen.

12. Verwendung von Sorbitanmonocaprylat zur Verbesserung der antimikrobiellen Wirksamkeit von Alkoholen der Formel (1)
R-OH (1)
worin R ein Rest bestehend aus Kohlenstoff-, Wasserstoff- und gegebenenfalls Sauerstoffatomen mit 5 - 12, vorzugsweise 6 - 11, Kohlenstoffatomen ist, und die Kohlenstoffatome untereinander linear, verzweigt und/oder zyklisch über gesättigte, ungesättigte und/oder aromatische Kohlenstoff-Kohlenstoff-Bindungen verknüpft sein können und die Reste auch Ethereinheiten enthalten können und wobei an die einzelnen Kohlenstoffatome Wasserstoffatome und/oder Hydroxygruppen gebunden sein können, **dadurch gekennzeichnet, dass** die Alkohole der Formel (1) aus aromatischen Alkoholen ausgewählt sind.

## Claims

1. A liquid composition containing
a) from 5 to 95% by weight, preferably from 10 to 90% by weight, particularly preferably from 20 to 80% by weight and especially preferably from 30 to 70% by weight, of sorbitan monocaprylate and
b) from 5 to 95% by weight, preferably from 10 to 90% by weight, particularly preferably from 20 to 80% by weight and especially preferably from 30 to 70% by weight, of one or more alcohols of the formula (1)
R-OH (1)
wherein R is a radical consisting of carbon, hydrogen and optionally oxygen atoms having 5 - 12, preferably 6 - 11, carbon atoms, and the carbon atoms can be linked to one another in a linear, branched and/or cyclic manner by means of saturated, unsaturated and/or aromatic carbon-carbon bonds and the radicals can also contain ether units and in which hydrogen atoms and/or hydroxyl groups can be bonded to the individual carbon atoms.

2. A liquid composition as claimed in claim 1, **characterized in that** the one or the more alcohols of the formula (1) are selected from the group consisting of aromatic alcohols, alkanediols and alkanetriols, and are preferably selected from the group consisting of aromatic alcohols and alkanediols.

3. A liquid composition as claimed in claim 1 or 2, **characterized in that** the one or the more alcohols of the formula (1) are selected from the group of alcohols consisting of benzyl alcohol, phenoxyethanol, propylene phenoxyethanol, phenethyl alcohol, 1,2-pentanediol, 1,2-hexanediol, 1,6-hexanediol, 1,2-octanediol, 1,2-decanediol, methylpropanediol and ethylhexylglycerol, wherein, among the aromatic alcohols, benzyl alcohol and phenoxyalcohol are in turn preferred and benzyl alcohol is particularly preferred.

4. A liquid composition as claimed in one or more of claims 1 to 3, **characterized in that** component b) comprises one or more alcohols of the formula (1) selected from aromatic alcohols, preferably 50% by weight, particularly preferably 60 to 100% by weight and especially preferably 100% by weight of one or more alcohols of the formula (1) selected from aromatic alcohols, the one or more aromatic alcohols being selected preferably from benzyl alcohol, phenoxyethanol, propylene phenoxyethanol and phenethyl alcohol and particularly preferably from benzyl alcohol and phenoxyalcohol, and the one or more aromatic alcohols especially preferably being benzyl alcohol.

5. A liquid composition as claimed in claim 4, **characterized in that** component b), in addition to the one or more alcohols of the formula (1) selected from aromatic alcohols, comprises one or more nonaromatic alcohols, preferably selected from the group of the alkanediols consisting of 1,2-hexanediol, 1,6-hexanediol, 1,2-octanediol, 1,2-decanediol and ethylhexylglycerol.

6. A liquid composition as claimed in one or more of claims 1 to 5, **characterized in that** it contains one or more other substances selected from
d) water,
e) antimicrobial active ingredients and
f) hydrotropes,
in which the antimicrobial active ingredients and hydrotropes are different to the alcohols of the formula (1).

7. A liquid composition as claimed in one or more of claims 1 to 6, **characterized in that** it contains one or more antimicrobial active ingredients that are different to the alcohols of the formula (1), in an amount from 0.5 to 30% by weight and preferably from 1.0 to 25% by weight.

8. A liquid composition as claimed in one or more of claims 1 to 7, **characterized in that** it contains one or more other additives.

9. A liquid composition as claimed in one or more of claims 1 to 8, **characterized in that** it has a clear appearance.

10. The use of a liquid composition as claimed in one or more of claims 1 to 9 for preserving cosmetic, dermatological or pharmaceutical products, preferably creams, cream gels, lotions, shampoos, shower baths, deodorants, antiperspirants, wet wipes, sunscreen formulations or decorative cosmetic articles.

11. The use of a liquid composition as claimed in one or more of claims 1 to 9 for the production of cosmetic, dermatological or pharmaceutical products, preferably of cosmetic, dermatological or pharmaceutical formulations.

12. The use of sorbitan monocaprylate for improving the antimicrobial efficacy of alcohols of the formula (1)
R-OH (1)
wherein R is a radical consisting of carbon, hydrogen and optionally oxygen atoms having 5 - 12, preferably 6 - 11, carbon atoms, and the carbon atoms can be linked to one another in a linear, branched and/or cyclic manner by means of saturated, unsaturated and/or aromatic carbon-carbon bonds and the radicals can also contain ether units and in which hydrogen atoms and/or hydroxyl groups can be bonded to the individual carbon atoms, **characterized in that** the alcohols of the formula (1) are selected from aromatic alcohols.

## Revendications

1. Composition liquide, contenant :
a) de 5 à 95 % en poids, de préférence de 10 à 90 % en poids, de manière particulièrement préférée de 20 à 80 % en poids et de manière notamment préférée de 30 à 70 % en poids de monocaprylate de sorbitane, et
b) de 5 à 95 % en poids, de préférence de 10 à 90 % en poids, de manière particulièrement préférée de 20 à 80 % en poids et de manière notamment préférée de 30 à 70 % en poids d'un ou de plusieurs alcools de formule (1)
R-OH (1)
dans laquelle R est un radical constitué d'atomes de carbone, d'hydrogène et éventuellement d'oxygène contenant 5 à 12, de préférence 6 à 11, atomes de carbone, et les atomes de carbone peuvent être reliés les uns avec les autres de manière linéaire, ramifiée et/ou cyclique par des liaisons carbone-carbone saturées, insaturées et/ou aromatiques, et les radicaux peuvent également contenir des unités éther, et des atomes d'hydrogène et/ou des groupes hydroxy peuvent être reliés aux atomes de carbone individuels, **caractérisée en ce que** le composant b) contient au moins 50 % en poids d'un ou de plusieurs alcools de formule (1) choisis parmi les alcools aromatiques.

2. Composition liquide selon la revendication 1, **caractérisée en ce que** le ou les alcools de formule (1) sont choisis dans le groupe constitué par les alcools aromatiques, les alcanediols et les alcanetriols, et sont de préférence choisis dans le groupe constitué par les alcools aromatiques et les alcanediols.

3. Composition liquide selon la revendication 1 ou 2, **caractérisée en ce que** le ou les alcools de formule (1) sont choisis dans le groupe des alcools constitué par l'alcool benzylique, le phénoxyéthanol, le propylène phénoxyéthanol, l'alcool phénéthylique, le 1,2-pentanediol, le 1,2-hexanediol, le 1,6-hexanediol, le 1,2-octanediol, le 1,2-décanediol, le méthylpropanediol et l'éthylhexylglycérine ; l'alcool benzylique et le phénoxyalcool étant préférés parmi les alcools aromatiques et l'alcool benzylique étant particulièrement préféré.

4. Composition liquide selon une ou plusieurs des revendications 1 à 3, **caractérisée en ce que** le composant b) contient 60 à 100 % en poids, et de préférence 100 % en poids, d'un ou de plusieurs alcools de formule (1) choisis parmi les alcools aromatiques, le ou les alcools aromatiques étant de préférence choisis parmi l'alcool benzylique, le phénoxyéthanol, le propylène phénoxyéthanol et l'alcool phénéthylique, et de manière particulièrement préférée parmi l'alcool benzylique et le phénoxyalcool, et le ou les alcools aromatiques étant de manière notamment préférée l'alcool benzylique.

5. Composition liquide selon une ou plusieurs des revendications 1 à 4, **caractérisée en ce que** le composant b) contient, en plus du ou des alcools de formule (1) choisis parmi les alcools aromatiques, un ou plusieurs alcools non aromatiques, de préférence choisis dans le groupe des alcanediols constitué par le 1,2-hexanediol, le 1,6-hexanediol, le 1,2-octanediol, le 1,2-décanediol et l'éthylhexylglycérine.

6. Composition liquide selon une ou plusieurs des revendications 1 à 5, **caractérisée en ce qu'**elle contient une ou plusieurs substances supplémentaires choisies parmi
d) l'eau,
e) les agents actifs antimicrobiens et
f) les hydrotropes,
les agents actifs antimicrobiens et les hydrotropes étant différents des alcools de formule (1).

7. Composition liquide selon une ou plusieurs des revendications 1 à 6, **caractérisée en ce qu'**elle contient un ou plusieurs agents actifs antimicrobiens, qui sont différents des alcools de formule (1), en une quantité de 0,5 à 30 % en poids et de préférence de 1,0 à 25 % en poids.

8. Composition liquide selon une ou plusieurs des revendications 1 à 7, **caractérisée en ce qu'**elle contient un ou plusieurs additifs supplémentaires.

9. Composition liquide selon une ou plusieurs des revendications 1 à 8, **caractérisée en ce qu'**elle présente une apparence limpide.

10. Utilisation d'une composition liquide selon une ou plusieurs des revendications 1 à 9 pour la conservation de produits cosmétiques, dermatologiques ou pharmaceutiques, de préférence de crèmes, de crèmes gels, de lotions, de shampoings, de bains douches, de déodorants, d'antiperspirants, de lingettes humides (wet wipes), de formulations de protection contre le soleil et d'articles cosmétiques décoratifs.

11. Utilisation d'une composition liquide selon une ou plusieurs des revendications 1 à 9 pour la fabrication de produits cosmétiques, dermatologiques ou pharmaceutiques, de préférence de formulations cosmétiques, dermatologiques ou pharmaceutiques.

12. Utilisation de monocaprylate de sorbitane pour améliorer l'efficacité antimicrobienne d'alcools de formule (1)
R-OH (1)
dans laquelle R est un radical constitué d'atomes de carbone, d'hydrogène et éventuellement d'oxygène contenant 5 à 12, de préférence 6 à 11, atomes de carbone, et les atomes de carbone peuvent être reliés les uns avec les autres de manière linéaire, ramifiée et/ou cyclique par des liaisons carbone-carbone saturées, insaturées et/ou aromatiques, et les radicaux peuvent également contenir des unités éther, et des atomes d'hydrogène et/ou des groupes hydroxy peuvent être reliés aux atomes de carbone individuels, **caractérisée en ce que** les alcools de formule (1) sont choisis parmi les alcools aromatiques.
